# EUROPEAN PATENT APPLICATION

(11) **EP 2 628 734 A2**
(43) Date of publication of application: **21.08.2013**
(21) Application number: 11832824.4
(22) Date of filing: 13.10.2011
(51) Int. Cl.: C07D 285/08

(54) **5-AMINO-1,2,4-THIADIAZOLE DERIVATIVES**

(30) Priority: 15.02.2011 RU 2011105263; 15.10.2010 RU 2010142209
(71) Applicant: Federalnoe Gosudarstvennoe Bydzhetnoe Uchrezhdenie Nauki Institut Fiziologicheski Aktivnikh Veschestv Rossiiskoi Akademii Nauk (IFAV RAN), Moskovskaya obl. 142432 (RU)
(72) Inventor: PROSHIN, Aleksey Nikolayevich, Moskovskaya obl. 142432 (RU); SERKOV, Igor Viktorovich, Moskovskaya obl. 142432 (RU); BACHURIN, Sergey Olegovich, Moskovskaya obl. 142432 (RU)
(74) Representative: King, Lawrence
(86) International application number: PCT/RU2011/000799
(87) International publication number: WO 2012/050484

(57) **Abstract**

The present invention relates in general to the field of organic chemistry and, in particular, to novel biologically active compounds of 5-amino[1,2,4]thiadiazole derivatives of the general formula: 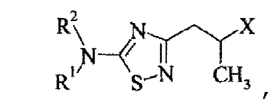 wherein
X is ONO₂ or NHR³;
R¹, R², and R³ may be the same or different and independently represent hydrogen, alkyl, aryl, aralkyl, heteroaryl, heteroaralkyl, cycloalkyl, as well as R¹ together with R² may represent heterocyclyl (for example, optionally substituted pyrrolidine, piperidine, azepane, piperazine, morpholine, etc.) provided that, when R¹ is H, then R² is other than hydrogen or methyl.
5-Amino-[1,2,4]thiadiazole derivatives are of special interest as drug candidates for prevention and treatment of neurodegenerative diseases, for example, Alzheimer's disease.

## Description

The invention relates to the field of organic chemistry and, in particular, to novel biologically active compounds of 5-amino[1,2,4]thiadiazole derivatives of the general formula: 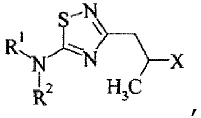 wherein
X is ONO₂ or NHR³;
R¹, R², and R³ may be the same or different and independently represent hydrogen, alkyl, aryl, aralkyl, heteroaryl, heteroaralkyl, cycloalkyl, as well as R¹ and R² taken together may represent heterocyclyl (for example, optionally substituted pyrrolidine, piperidine, azepane, piperazine, morpholine, etc.), provided that when R¹ is H, then R² is other than hydrogen or methyl.

The term "alkyl" refers to an unsubstituted or substituted, straight-chain or branched-chain alkyl group having 1 to 12 carbon atoms. The alkyl group can be substituted with halogens (for example, fluorine, chlorine, etc.), alkoxy groups (for example, methoxy, ethoxy, etc.), cyano, nitro, trihalomethyl groups (for example, trifluoromethyl, etc.), optionally substituted amino groups (for example, amino, dimethylamino, acetylamino, N-piperidino, etc.), acyl groups (for example, formyl, acetyl, benzoyl, etc.), carboxamide groups (for example, N,N-diethylcarboxamide group, etc.), carboxyl groups, carbalkoxy groups, etc.

The term "cycloalkyl" refers to a cyclic saturated hydrocarbon group having 3 to 8 ring carbon atoms (for example, cyclopropyl, cyclohexyl, etc.).

The term "aryl" refers to an unsubstituted or substituted phenyl group. The phenyl group can be substituted with halogens (for example, fluorine, chlorine, etc.), lower alkyl groups (for example, methyl, ethyl, etc.), lower alkoxy groups (for example, methoxy, ethoxy, etc.), cyano, nitro, trihalomethyl groups (for example, trifluoromethyl, etc.), optionally substituted amino groups (for example, amino, dimethylamino, acetylamino, N-piperidino, etc.), acyl groups (for example, formyl, acetyl, benzoyl, etc.), carboxamide groups (for example, N,N-diethylcarboxamide group, etc.), carboxyl groups, carbalkoxy groups, etc.

The term "aralkyl" refers to the above-defined aryl to which the above-defined alkyl group is attached.

The term "heteroaryl" refers to a N-, O-, or S-containing 5-or 6-membered, heteroaromatic cycle. Examples of suitable substituents include optionally substituted furan, thiophene, pyrrole, indole, pyridine, quinolone, etc.

The term "heteroaralkyl" refers to the above-defined heteroaryl to which the above-defined alkyl group is attached. Examples of suitable substituents include optionally substituted ethylpiperazine-1-yl, ethylindol-3-yl, propyl-3-imidazol-1-yl, etc.

The term "halogen" refers to chlorine, fluorine, bromine, or iodine.

The term "alkoxy" means an Alk-O group, wherein the alkyl moiety is the same as the above-defined alkyl group (for example, methoxy, ethoxy, etc.).

Recently, construction of 5-membered heterocyclic skeletons serving as an ideal scaffold pharmacophore demonstrating a wide range of biological activities, is used to develop new drugs [V. Krchnak et al., Chem. Rev., 2002, 102, 61]. In this respect, a thiadiazole cycle is one of a preferable structure to design libraries of new "drug-like" compounds [M. Pal et al., J. Org. Chem., 2003, 68, 6806]. In particular, [1,2,4]thiadiazole derivatives are used as a major scaffold for drugs of different therapeutic activity, such as anti-inflammatory [Y. Song et al., J. Bioorg. Med. Chem., 2004, 12, 5107], anticonvulsive [T. Akbarzadeh et al., J. Bioorg. Med. Chem., 2003, 11, 769], antihypertensive [Tyagi M. et al., Oriental J. Chem., 2002, 18, 125], neuroprotective [A. Gupta et al., J. Med. Chem., 2009, 44, 1100] activity.

5-Amino-[1,2,4]thiadiazole derivatives are an object of a special interest as drugs for the prevention and treatment of neurodegenerative diseases, for example, Alzheimer's disease [A. Castro et al., J. Bioorg. Med. Chem., 2006, 14, 1644]. These compounds are described as able to prevent aggregation of the amyloid-β peptide into specific senile plaques that are known to be major neuropathologic characteristics of Alzheimer's disease [K. Baumann et al. "Modulators for Amyloid beta", US Patent Application Publication 20090215759 A1]. Thus, one of the perspective directions in the medicinal chemistry is the development of hybrid multi-targeted drugs by introducing a moiety being a nitric acid (NO) generator, into a known drug molecule [I.V. Serkov, V.V. Bezuglov, Uspekhi khimii, 2009, 78, 442-465]. NO is an endogen signal molecule with a wide range of biological activity, which plays an important role in the nerve functions. In this connection, NO-donors are used in the treatment of various degenerative diseases, including the Alzheimer's disease therapy [G.R.J. Thatcher, B.M. Bennett, J.N. Reynolds, Curr. Alzheimer Res., 2005, 2, 171-182]. A NO-generating moiety is also introduced into known drugs useful for this purpose, for example, tacrine-containing NO-donor drugs [L. Fang et al., J. Med. Chem., 2008, 51, 713-716].

The proposed 5-amino-[1,2,4]thiadiazole derivatives, in particular 5-amino-3-(2-aminopropyl)-1,2,4-thiadiazole compounds of general formula **6** and 5-amino-3-(2-nitroxypropyl)-1,2,4-thiadiazole compounds of general formula **8** can be prepared according to the following scheme.

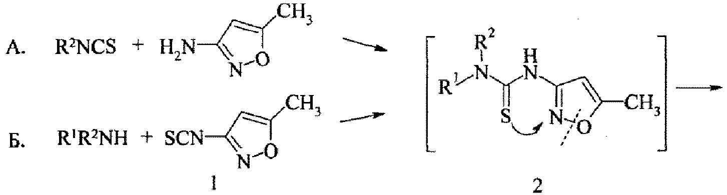

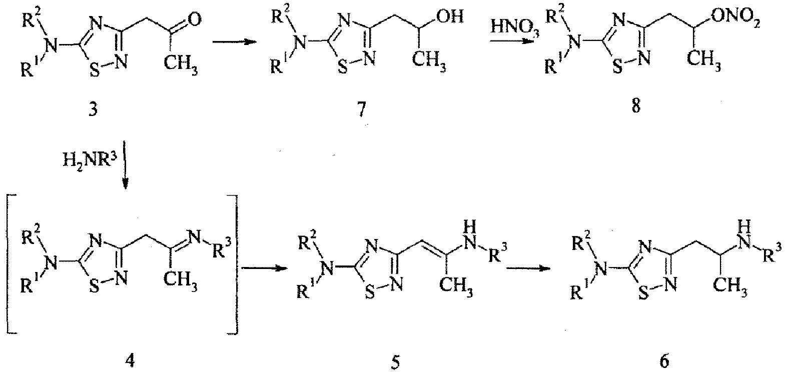

5-amino-3-(2-oxopropyl)-[1,2,4]-thiadiazole **3** used as an initial compound for the claimed compounds, can be synthesized in two ways.

Method **A.** 5-N-Monosubstituted 5-amino-3-(2-oxopropyl)-[1,2,4]thiadiazole compounds of general formula **3** (R¹ = H) were obtained through the reaction of isothiocyanate R²NCS with 3-amino-5-methylisoxazole. Isoxazole-substituted thiourea **2** formed through the Boulton-Katrizky rearrangement is recycled in situ into 5-amino-3-(2-oxopropyl)-[1,2,4]-thiadiazole **3** [A.J. Boulton, A.R. Katrizky, A.M. Hamid, J. Chem. Soc., 1967, 2005].

Method **B**. 5-N,N-Disubstituted amino-3-(2-oxopropyl)-[1,2,4]thiadiazole compounds of general formula **3** were obtained through the reaction of 5-methylisoxazolyl-3-isothiocyanate **1** with the corresponding secondary amine R¹R²NH.

In order to prepare amino-derivatives of [1,2,4]thiadiazole **6,** 5-amino-3-(2-oxopropyl)-[1,2,4]-thiadiazole **3** was condensed in methanol with a primary amine H₂NR³. The imine **4** which is formed in the reaction, rearranged into the corresponding 5-amino-3-(2-amino-1-propenyl)-[1,2,4]-thiadiazole **5,** reduction thereof with sodium borohydride allows to obtain the target 5-amino-3-(2-oxypropyl)-[1,2,4]-thiadiazole **6.**

Then a reduction was conducted of keto group of 5-amino-3-(2-oxopropyl)-[1,2,4]-thiadiazole **3** with sodium borohydride to obtain nitroxy derivatives of [1,2,4]thiadiazole **8**. The 5-amino-3-(2-oxypropyl)-[1,2,4]-thiadiazole **7** thus obtained was reacted with concentrated nitric acid to obtain the target 5-amino-3-(2-nitroxypropyl)-[1,2,4]-thiadiazole **8**.

The following examples are provided to illustrate the present invention, without any limitation of the scope thereof.

**Example 1.** Synthesis of 5-(3,4-dichlorophenylamino)-3-(2-nitroxypropyl)-[1,2,4]-thiadiazole.

A solution of 3,4-dicholorophenyl isothiocyanate (2.04 g, 0.01 M) in 10 ml of acetonitrile was added dropwise under stirring to a solution of 3-amino-5-methylisoxazole (0.98 g, 0.01 M) and 50 mg of p-toluenesulfonic acid in 20 ml of acetonitrile. After the dropwise addition, the mixture was heated to reflux temperature and then kept at room temperature, until 3,4-dichlorophenyl-[3-(2-oxopropyl)-[1,2,4]-thiadiazol-5-yl]-amine precipitated, than the obtained product was filtered. The obtained oxothiadiazole (3.02 g, 0.01 M) was suspended in 30 ml of methanol, heated to 50°C, and sodium borohydride (0.38 g, 0.01 M) was added portionwise under vigorous stirring. After end of the reaction, methanol was evaporated, 50 ml of methylene chloride was added, the resultant mixture was washed with water (2x50 ml), and the organic layer was separated and dried over sodium sulfate. The drying agent was filtered off, and the filtrate was evaporated to obtain 3,4-dichlorophenyl-[3-(2-oxypropyl)-[1,2,4]-thiadiazol-5-yl]-amine. The obtained hydroxythiadiazole (0.304 g, 0.001 M) was dissolved in 1 ml of methylene chloride and added under stirring to a solution of 500 µl of concentrated nitric acid in 5 ml of methylene chloride, wherein the solution was cooled to 0°C. The reaction mixture was stirred for 40 minutes, washed with water (3x10 ml), and the organic layer was dried over sodium sulfate. The drying agent was filtered off, and the filtrate was evaporated to obtain 5-(3,4-dichlorophenylamino)-3-(2-nitroxypropyl)-[1,2,4]-thiadiazole. Light brown crystals. M.p. = 118-120°C. ¹H NMR (CDCl₃), δ ppm: 8.55 (1H, brs, NH), 7.28 (3H, m, H_{arom.}), 5.59 (1H, m, CH), 3.10 (2H, m, CH₂), 1.45 (3H, d, J=6.2 Hz, CH₃).

5-(3-tolylamino)-3-(2-nitroxypropyl)-[1,2,4]-thiadiazole was obtained in the same way from 3-methylaniline. Yellow crystals. M.p. = 102-104°C. ¹H NMR (CDCl₃), δ ppm: 8.12 and 7.31 (4H, m, H_{arom.}), 5.65 (1H, m, CH), 3.16 (2H, m, CH₂), 2.66 (3H, s, CH₃), 1.45 (3H, d, J=6.2 Γ 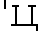 , CHCH₃).

5-Phenylamino-3-(2-nitroxypropyl)-[1,2,4]-thiadiazole was obtained in the same way from aniline. Yellow crystals. M.p. = 72-74°C. ¹H NMR (CDCl₃), δ ppm: 11.02 (1H, s, NH), 8.26 (2H, d, J=9.2 Hz, H_{arom.}), 7.73 (2H, d, J=9.2 Hz, H_{arom.}), 7.36 (1H, s, H_{arom.}), 5.71 (1H, m, CH); 3.22 (2H, m, CH₂), 1.54 (3H, d, J=6.2 Hz, CHCH₃).

5-(2-Chloro-5-trifluoromethylphenylamino)-3-(2-nitroxypropyl)-[1,2,4]-thiadiazole was obtained in the same way from 2-chloro-5-trifluoromethylaniline. Light yellow crystals. M.p. = 96-97°C. ¹H NMR (CDCl₃), δ ppm: 8.28 (1H, m, H_{arom.}), 8.06 (1H, s, NH), 7.49 (1H, dm, J=8.4 Hz, H_{arom.}), 7.27 (1H, m, H_{arom.}), 5.60 (1H, m, CH), 3.15 (2H, m, CH₂), 1.48 (3H, d, J=6.4 Hz, CHCH₃).

5-(2-Methyl-3-chlorophenylamino)-3-(2-nitroxypropyl)-[1,2,4]-thiadiazole was obtained in the same way from 2-methyl-3-chloroaniline. Dark yellow oil. ¹H NMR (CDCl₃), δ ppm: 9.56 (1H, s, NH), 7.81 (1H, m, H_{arom.}), 7.22 (2H, m, H_{arom.}), 5.58 (1H, m, CH), 3.18 (2H, m, CH₂), 1.47 (3H, d, J=6.4 Hz, CHCH₃).

5-Cyclopropylamino-3-(2-nitroxypropyl)-[1,2,4]-thiadiazole was obtained in the same way from cyclopropylamine. Dark yellow oil. ¹H NMR (DMSO-d₆), δ ppm: 8.67 (1H, s, NH), 5.53 (1H, m, CH), 4.10 (1H, m, CH), 3.12 (2H, m, CH₂), 1.42 (3H, d, J=6.2 Hz, CHCH₃), 0.71 (4H, m, CH₂CH₂).

### Example 2. Synthesis of [3-(2-nitroxypropyl)-[1,2,4]-thiadiazol-5-yl]-piperidine.

A solution of 5-methylisoxazolyl-3-isothiocyanate (1.4 g, 0.01 M) in 10 ml of acetonitrile was added dropwise under stirring to a solution of piperidine (0.85 g, 0.01 M) and 50 mg of p-toluenesulfonic acid in 20 ml of acetonitrile. After the dropwise addition, the mixture was heated to reflux temperature and then kept at room temperature, until precipitation of [3-(2-oxopropyl)-[1,2,4]-thiadiazol-5-yl]-piperidine which was filtered off. The obtained oxothiadiazole (2.25 g, 0.01 M) was suspended in 30 ml of methanol, heated to 50°C, and sodium borohydride (0.38 g, 0.01 M) was added portionwise under vigorous stirring. After end of the reaction, methanol was evaporated, 50 ml of methylene chloride was added, the resultant mixture was washed with water (2x50 ml), and the organic layer was separated and dried over sodium sulfate. The drying agent was filtered, the filtrate was evaporated and [3-(2-oxypropyl)-[1,2,4]-thiadiazol-5-yl]-piperidine was obtained. The obtained oxythiadiazole (0.227 g, 0.001 M) was dissolved in 1 ml of methylene chloride and added under stirring to a solution of 500 µl of concentrated nitric acid in 5 ml of methylene chloride, wherein the solution was cooled to 0°C.

The reaction mixture was stirred for 40 minutes, washed with water (3x10 ml), and the organic layer was dried over sodium sulfate. The drying agent was filtered, and the filtrate was evaporated to obtain [3-(2-nitroxypropyl)-[1,2,4]-thiadiazol-5-yl]-piperidine. Yellow oil. ¹H NMR (CDCl₃), δ ppm: 5.63 (1H, m, CH), 3.48 (4H, brs, 2xNCH₂), 3.11 (1H, dd, J=6.8 Hz, J=14.9 Hz, CHH), 2.94 (1H, dd, J=6.6 Hz, J=14.9 Hz, CHH), 1.70 (6H, brs, CH₂CH₂CH₂), 1.44 (3H, d, J=6.4 Hz, CHCH₃).

1-[3-(2-Nitroxypropyl)-[1,2,4]-thiadiazol-5-yl]-4-pyridin-2-yl-piperazine was obtained in the same way from 1-pyridin-2-yl-piperazine. Dark yellow oil. ¹H NMR (CDCl₃), δ ppm: 8.28 (1H, m, H_{arom.}), 7.56 (1H, m, H_{arom.}), 6.77 (1H, m, H_{arom.}), 6.70 (1H, m, H_{arom.}), 5.55 (1H, m, CH), 3.73 (4H, m, N(CH₂CH₂)₂N-αPy), 3.63 (4H, m, N(CH₂CH₂)₂N-αPy), 3.14 (2H, m, CH₂), 1.44 (3H, d, J=6.4 Hz, CHCH₃).

1-[3-(2-Nitroxypropyl)-[1,2,4]-thiadiazol-5-yl]-dibenzylamine was obtained in the same way from dibenzylamine. Yellow oil. ¹H NMR (CDCl₃), δ ppm: 7.58 (10H, m, H_{arom.}), 5.58 (1H, m, CH), 4.64 (4H, s, N(CH₂)₂), 3.10 (2H, m, CH₂), 1.40 (3H, d, J=6.2 Hz, CHCH₃).

1-[3-(2-Nitroxypropyl)-[1,2,4]-thiadiazol-5-yl]-5-methyl-isoxazol-3-ylamine was obtained in the same way from 3-amino-5-methylisoxazole. Yellow oil. ¹H NMR (CDCl₃), δ ppm: 6.01 (1H, s, =CH), 5.56 (1H, m, CH), 3.13 (2H, m, CH₂), 2.41 (3H, s, CH₃), 1.42 (3H, d, J=6.2 Γ 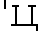 , CHCH₃).

5-(6-Methyl-pyridin-2-ylamino)-3-(2-nitroxypropyl)-[1,2,4]-thiadiazole was obtained in the same way from 2-amino-5-methylpyridine. Dark yellow oil. ¹H NMR (CDCl₃), δ ppm: 10.05 (1H, s, NH), 7.62 (1H, t, J=7.8 Hz, H_{arom.}), 6.87 (1H, d, J=7.6 Γ 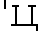 , H_{arom.}), 6.79 (1H, d, J=7.8 Γ 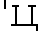 , H_{arom.}), 5.61 (1H, m, CH), 3.22 (2H, m, CH₂), 1.49 (3H, d, J=6.5 Hz, CHCH₃).

### Example 3. {2-[5(3-Chloro-4-methyl-phenylamino)-[1,2,4]thiadiazol-3-yl]-1-methyl-ethyl}-(2,2,6,6-tetramethyl-piperidin-4-yl)-amine (method A).

A solution of 3-amino-5-methylisoxazole (4.9 g, 0.05 M) in 50 ml of acetonitrile was added dropwise under stirring to a solution of 3-chloro-4-methylphenylisothiacyanate (9.17 g, 0.05 M) and 50 mg of p-toluenesulfonic acid in 50 ml of acetonitrile. After the dropwise addition, the mixture was heated to reflux temperature and then kept at room temperature, until precipitation, and the precipitate was filtered and recrystallized from isopropanol (30 ml).

The obtained oxothiadiazole (5.62 g, 0.02 M) and 4-amino-2,2,6,6-tetramethylpiperidine (3.12 g, 0.02 M) were dissolved in 10 ml of methanol and kept at room temperature for a day. The resulting precipitate was filtered off.

The yield of {2-[5-(3-chloro-4-methyl-phenylamino)-[1,2,4]thiadiazol-3-yl]-1-methyl-vinyl}-(2,2,6,6)-tetramethyl-piperidin-4-yl)-amine was 7.72 g (82% based on the initial isothiocyanate). Colorless crystals. M.p. = 70-72°C. ¹H NMR (CDCl₃), δ ppm: 8.19 (1H, d, J=8.6 Hz, NH), 7.33 (1H, d, J=2.3 Hz, H_{arom.}), 7.26 (1H, d, J=8.4 Hz, H_{arom.}), 7.10 (1H, dd, J=2.3, 8.1 Hz, H_{arom.}), 5.11 (1H, s, =CH), 3.78 (1H, m, NCH), 2.39 (3H, s, Ph-CH₃), 2.08 (3H, s, =C-CH₃), 1.96 (2H, dd, J=3.5, 12.8 Hz, 2CH), 1.28 (6H, s, 2CH₃), 1.18 (6H, s, 2CH₃), 1.16 (1H, brs, NH), 1.11 (2H, t, J=12.3 Hz, 2CH).

{2-[5-(3-chloro-4-methyl-phenylamino)-[1,2,4]thiadiazol-3-yl]-1-methyl-vinyl}-(2,2,6,6)-tetramethyl-piperidin-4-yl)-amine (4.20 g, 0.01 M) was suspended in 30 ml of methanol, heated to 50°C, and sodium borohydride (0.38 g, 0.01 M) was added portionwise under vigorous stirring. After end of the reaction, methanol was evaporated, 50 ml of methylene chloride was added, the resultant mixture was washed with water (2x50 ml), and the organic layer was separated and dried over sodium sulfate. The drying agent was filtered off, the filtrate was evaporated and the yield of {2-[5-(3-chloro-4-methyl-phenylamino)-[1,2,4]thiadiazol-3-yl]-1-methyl-ethyl}-(2,2,6,6-tetramethyl-piperidin-4-yl)-amine was 3.74 (89%). Colorless crystals. M.p. = 76-78°C. ¹H NMR (DMSO-d₆), δ ppm: 7.71 (1H, d, J=2.2 Hz, H_{arom.}), 7.29 (1H, dd, J=2.2, 8.4 Hz, H_{arom.}), 7.17 (1H, d, J=8.4 Hz, H_{arom.}), 3.32 (1H, m, NCH), 2.89 (1H, tt, J=3.5, 11.6 Hz, NCH), 2.71 (2H, dd, J=6.3, 14.2 Hz, CH₂), 2.31 (3H, s, Ph-CH₃), 1.71 (2H, dd, J=3.5, 12.3 Hz, 2CH), 1.10 (6H, d, J=2.8 Hz, (CH₃)₂), 1.06 (3H, d, J=6.3 Hz, CCH₃), 1.00 (6H, d, J=5.8 Hz, (CH₃)₂), 0.75 (H, t, J=12.0 Hz, CHH), 0.65 (H, t, J=12.0 Hz, CHH).

**Example 4.** [5-Azepan-1-yl-[1,2,4]thiadiazol-3-yl)-1-methyl-vinyl]-(2,2,6,6-tetramethyl-piperidin-4-yl)-amine (Method **B**)**.**

5-Methylisoxazolyl-3-isothiocyanate **1.**

A solution of 49.05 g (0.5 M) of 3-amino-5-methylisoxazole in 300 ml of methylene chloride was added dropwise under vigorous stirring to a mixture of 42.2 ml (0.55 M) of thiophosgene, 92.4 g (1.1 M) of sodium bicarbonate in 200 MJI of water, wherein the mixture was cooled to 0°C. After the dropwise addition, the mixture was stirred for additional 30 minutes. The organic phase was separated, washed with a saturated solution of sodium chloride (2x100 ml), dried over sodium sulfate and evaporated. The resulting oil was distilled, and the fraction was separated at temperature of 39-41°C under pressure of 1 mm Hg. The yield was 36.8 g (52.6%). ¹H NMR (CDCl₃, ppm, δ): 2.50 (3H, s, CH₃), 6.00 (1H, s, CH). ¹³C NMR (CDCl₃, ppm, δ): 172.9, 153.1, 145.4, 100.1, 13.2

A solution of hexamethyleneimine (0.99 g, 0.01 M) in 10 ml of acetonitrile was added dropwise under stirring to a solution of 5-methylisoxazolyl-3-isothiocyanate **1** (1.4 g, 0.01 M) and 50 mg of p-toluenesulfonic acid in 10 ml of acetonitrile. After the dropwise addition the mixture was heated to reflux temperature and then kept at room temperature, until precipitation of [3-(2-oxopropyl)-[1,2,4]-thiadiazol-5-yl]-azepane **3** which was filtered off. The obtained oxothiadiazole **3** (2.39 g, 0.01 M) and 4-amino-2,2,6,6-tetramethylpiperidine (1.70 g, 0.01 M) were dissolved in 10 ml of methanol and kept at room temperature for a day. The resulting precipitate was filtered. The yield of {2-[5-azepan-1-yl-[1,2,4]thiadiazol-3-yl)-1-methyl-vinyl}-(2,2,6,6-tetramethyl-piperidin-4-yl)-amine was 3.86 g (78%). Yellow crystals. M.p.= 86-88°C. ¹H NMR (CDCl₃), δ ppm: 8.22 (1H, d, J=8.7 Hz, NH), 5.10 (1H, s, =CH), 3.74 (1H, m, NCH), 2.06 (3H, s, =C-CH₃), 1.96 (2H, dd, J=3.5, 12.8 Hz, 2CH), 1.88 (4H, m, CH₂NCH₂), 1.64 (8H, m, (CH₂)₄), 1.28 (6H, s, 2CH₃) 1.18 (6H, s, 2CH₃), 1.16 (1H, brs, NH), 1.07 (2H, t, J=12.2 Hz, 2CH).

The obtained {2-[5-azepan-1-yl-[1,2,4]thiadiazol-3-yl)-1-methyl-vinyl}-(2,2,6,6-tetramethyl-piperidin-4-yl)-amine **5** (3.91 g, 0.01 M) was suspended in 30 ml of methanol, heated to 50°C and sodium borohydride (0.38 g, 0.01 M) was added portionwise under vigorous stirring. After end of the reaction, methanol was evaporated, 50 ml of methylene chloride was added, the resultant mixture was washed with water (2x50 ml), and the organic layer was separated and dried over sodium sulfate. The drying agent was filtered off, the filtrate was evaporated, and [5-azepan-1-yl-[1,2,4]thiadiazol-3-yl)-1-methyl-vinyl]-(2,2,6,6-tetramethyl-piperidin-4-yl)-amine **6** was obtained. The yield was 3.32 g (85%). Oil. ¹H NMR (DMSO-d₆), δ ppm: 3.26 (1H, m, NCH), 2.88 (1H, tt, J=3.5, 11.6 Hz, NCH), 2.71 (2H, dd, J=6.3, 14.2 Hz, CH₂), 1.83 (4H, m, CH₂NCH₂), 1.73 (2H, dd, J=3.5, 12.3, 2CH), 1.63 (8H, m, (CH₂)₄), 1.12 (6H, d, J=2.3 Hz, (CH₃)₂), 1.03 (3H, d, J=6.3 Hz, CCH₃), 1.03 (6H, d, J=2.6 Hz, (CH₃)₂), 0.75 (1H, t, J=12.1 Hz, CHH), 0.65 (1H, t, J=12.1 Hz, CHH).

The following derivatives of 5-amino-3-(2-aminopropyl)-[1,2,4]thiadiazole were obtained by method **A.**

### [3-(2-Benzylamino-propyl)-[1,2,4]thiadiazol-5-yl]-(3-chloro-4-fluoro-phenyl)-amine

Colorless crystals. M.p. = 77-79°C. ¹H NMR (CDCl₃), δ ppm: 10.80 (1H, brs, NH), 7,42 (1H, m, NH), 7,30 (5H, s, H_{arom.}) 7.17 (2H, m, H_{arom.}), 3.80 (2H, dd, J=12.2, CH₂), 3.28 (1H, m, CH), 2.94 (2H, dd, 14.2 Hz, CH₂), 1.21 (3H, d, J=6.3 Hz, CH₃).

### (3-Chloro-4-fluoro-phenyl)-{3[2-(2-morpholin-4-yl-ethylamino)-propyl]-[1,2,4]thiadiazol-5yl}-amine

Colorless crystals. M.p. = 108-110°C. ¹H NMR (DMSO-d₆), α ppm: 7.90 (1H, dd, J=2.2, 6.5 Hz, H_{arom.}), 7.45 (1H, m, H_{arom.}), 7.20 (1H, t, J=8.8 Hz, H_{arom.}), 3.50 (4H, t, J=4.7 Hz, CH₂OCH₂), 3.13 (1H, m, NCH), 2.77 (2H, dd, J=6.6, 14.2 Hz, CH₂), 2.65 (2H, q, J=5.6, Hz, NCH₂), 2.39 (2H, t, NCH₂), 2.30 (4H, t, J=4.7 Hz, CH₂NCH₂), 1.11 (3H, d, J=6.3 Hz, CH₃).

### (3-Chloro-4-fluoro-phenyl)-{3[2-(2-morpholin-4-yl-propylamino)-propyl]-[1,2,4]thiadiazol-5yl}-amine

Colorless crystals. M.p. = 72-74°C. ¹H NMR (CDCl₃), δ ppm: 7.37 (1H, m, H_{arom.}) 7.18 (1H, s, H_{arom.}), 7.10 (1H, m, H_{arom.}), 3.61 (4H, t, J=4.7 Hz, CH₂OCH₂), 3.15 (1H, m, NCH), 2.78 (2H, dd, J=7.1, 14.4 Hz, CH₂), 2.62 (2H, m, NCH₂), 2.33 (4H, t, J=4.7 Hz, CH₂NCH₂), 2.30 (2H, t, NCH₂), 1.59 (2H, m, NCH₂CH₂CH₂N), 1.06 (3H, d, J=6.4 Hz, CCH₃).

### [2-(5-Cyclopropylamino)-[1,2,4]thiadiazol-3-yl-1-methyl-ethyl]-(2,2,6,6-tetramethyl-piperidin-4-y1)-amine

Colorless crystals. M.p. = 75-77°C. ¹H NMR (CDCl₃), δ ppm: 3.28 (1H, m, NCH), 2.84 (1H, tt, J=3.6, 11.6 Hz, NCH), 2.68 (2H, d, J=6.2 Hz, CH₂), 2.54 (1H, m, NCH(CH₂)₂), 1.73 (2H, d, J=12.3 Hz, 2CH), 1.06 (6H, d, J=2.8 Hz (CH₃)₂), 1.04 (3H, d, J=6.3 Hz, CCH₃), 1.01 (6H, d, J=5.8 Hz, (CH₃)₂), 0.80-0.50 (6H, m, NCH (CH₂)₂, CHH).

### 2-{2-[5(4-Fluoro-phenylamino)-thiadiazol-3-yl]-1-methyl-ethylamino}-ethanol

Colorless crystals. M.p. = 97-99°C. ¹H NMR (DMSO-d₆), δ ppm: 7.56 (2H, dd, J=4.9, 9.1 Hz, 2H_{arom.}), 7.07 (2H, t, J=8.7 Hz, 2H_{arom.}), 4.20 (1H, brs, OH), 3.49 (2H, t, J=5.3 Hz, CH₂OH), 3.16 (1H, m, NCH), 2.75 (2H, dd, J=6.0, 14.0 Hz, CH₂), 2.65 (2H, q, J=6.0 Hz, NCH₂), 1.10 (3H, d, J=6.3 Hz, CH₃).

### (4-Chloro-phenyl)-[3-(2-methylamino-propyl)-[1,2,4]thiadiazol-5-yl]-amine

Colorless crystals. M.p. = 92-94°C. ¹H NMR (DMSO-d₆), δ ppm: 7.56 (2H, d, J=8.7 Hz, 2H_{arom.}), 7.20 (2H, d, J=8.7 Hz, 2H_{arom.}), 3.04 (1H, m, NCH), 2.75 (2H, dd, J=6.2, 14.1 Hz, CH₂), 2.35 (3H, s, NCH₃), 1.06 (3H, d, J=6.4 Hz, CH₃).

### 4-(2-{2-[5-(3-Chloro-4-methyl-phenylamino)-[2,4,4]thiadiazol-3-yl]-1-methyl-ethylamino}-ethyl)-phenylsulfamide

Colorless crystals. M.p. = 143-145°C. ¹H NMR (CD₃CN), δ ppm: 7.93 (1H, dd, J=2.8, 6.7 Hz, H_{arom.}), 7.78 (2H, d, J=8.2 Hz, 2H_{arom.}), 7.45 (1H, m, H_{arom.}), 7.39 (2H, d, J=8.2 Hz, 2H_{arom.}), 7.29 (1H, t, J=8.8 Hz, H_{arom.}), 3.26 (1H, m, NCH), 2.97 (1H, m, NCH), 2.85 (4H, m, NCH₂CH₂Ph), 1.15 (3H, d, J=6.3 Hz, CH₃).

Some derivatives of 5-amino-3-(2-aminopropyl)-[1,2,4]thiadiazole that were synthesized by method **B**.

### 2-[5-(Cyclopropyl-thiophen-2-ylmethyl-amino)-[1,2,4]thiadiazol-3-yl]-1-methyl-ethyl}-]-(2,2,6,6-tetramethyl-piperidin-4-yl)-amine

Oil. ¹H NMR (DMSO-d₆), δ ppm: 7.25 (1H, dd, J=1.4, 5.5 Hz, H_{arom.}), 7.06 (1H, dd, J=1.2, 3.5 Hz, H_{arom.}), 6.94 (1H, dd, J=3.5, 5.1 Hz, H_{arom.}), 4.95 (2H, s, CH₂), 3.29 (1H, m, NCH), 2.93 (1H, tt, J=3.5, 11.6 Hz, NCH), 2.70 (2H, dd, J=6.3, 14.2 Hz, CH₂), 2.64 (1H, m, NCH), 1.71 (2H, dd, J=3.5, 12.3 Hz, 2CH), 1.12 (6H, d, J=2.3 Hz, (CH₃)₂), 1.05 (3H, d, J=6.5 Hz, CCH₃), 1.03 (6H, d, J=4.0 Hz, (CH₃)₂), 0.91 (4H, m, (CH₂)₂), 0.76 (1H, t, J=12.1 Hz, CHH), 0.67 (1H, t, J=12.1 Hz, CHH)

### {1-Methyl-2-[5-(4-methyl-piperazin-1-yl)-[1,2,4]thiadiazol-3-yl]-ethyl}-(2,2,6,6-tetramethyl-piperidin-4-yl)-amine

Oil. ¹H NMR (DMSO-d₆), δ ppm: 3.54 (4H, t, J=5.1 Hz, CH₂NCH₂), 3.35 (1H, m, NCH), 2.95 (1H, tt, J=3.5, 11.6 Hz, NCH), 2.78 (2H, d, J=6.0, CH₂), 2.54 (4H, t, J=5.1 Hz, CH₂NCH₂), 2.36 (3H, s, CH₃), 1.83 (2H, dd, J=3.5, 12.3 Hz, 2CH) 1.18 (6H, d, J=2.3 Hz (CH₃)₂), 1.13 (3H, d, J=6.3 Hz, CCH₃), 1.10 (6H, d, J=2.6 Hz, (CH₃)₂), 0.80 (1H, t, J=12.1 Hz, CH), 0.70 (1H, t, J=12.1 Hz, CH).

### (2-{5-[(4-Dimethylamino-benzyl)-pyridin-3-ylmethyl-amino]-[1,2,4]thiadiazol-3-yl}-1-methyl-ethyl)-(2,2,6,6-tetramethyl-piperidin-4-yl)-amine

Oil. ¹H NMR (DMSO-d₆), δ ppm: 8.42 (2H, m, 2Py-α), 7.60 (H, dt, J=1.9, 7.9 Hz, Py-y), 7.25 (H, dd, J=0.7, 4.7, 7.7 Hz, Py-β), 7.04 (2H, d, J=8.8 Hz, 2H_{arom.}), 6.56 (2H, d, J=8.8 Hz, 2H_{arom.}), 4.67 (2H, s, NCH₂), 4.43 (2H, s, NCH₂), 3.33 (4H, q, CH₃CH₂NCH₂CH₃), 3.27 (1H, m, NCH), 2.91 (1H, tt, J=3.5, 11.6 Hz, NCH), 2.65 (2H, d, J=6.0 Hz, CH₂), 1.69 (2H, dd, J=3.5, 12.3, 2CH), 1.15 (6H, t, CH₃CH₂NCH₂CH₃), 1.12 (6H, d, J=4.2 Hz, (CH₃)₂), 1.05 (3H, d, J=6.3 Hz, CCH₃), 1.01 (6H, d, J=4.2 Hz, (CH₃)₂), 0.73 (1H, t, J=12.1 Hz, CH), 0.63 (1H, t, J=12.1 Hz, CH).

### Example 5. Neurotropic activity of 5-amino-3-(2-nitroxypropyl)-1,2,4-thiadiazole derivatives.

Neuroprotective action of new compounds was studied in P₂ synaptosomal fraction from rat brain cortex. 5-amino-3-(2-nitroxypropyl)-1,2,4-thiadiazole derivatives were studied in the test on glutamate-induced uptake of ⁴⁵Ca²⁺ isotope into synaptosomes. 1-[3-(2-Nitroxypropyl)-[1,2,4]-thiadiazol-5-yl]-4-pyridin-2-yl-piperazine at a concentration of 100 µM demonstrated 98% inhibition of the uptake of ⁴⁵Ca²⁺ into synaptosomes vs. a control (IC₅₀=16.6 MκM).

## Claims

1. Novel biologically active compounds - 5-amino[1,2,4]thiadiazole derivatives of the general formula: 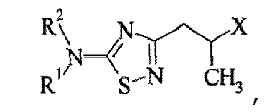 wherein X is ONO₂ or NHR³;
R¹, R², and R³ may be the same or different and independently represent hydrogen, alkyl, aryl, aralkyl, heteroaryl, heteroaralkyl, cycloalkyl, as well as R¹ together with R² may represent heterocyclyl (for example, optionally substituted pyrrolidine, piperidine, azepane, piperazine, morpholine, etc.) provided that, when R¹ is H, then R² is other than hydrogen or methyl.

2. The compounds according to claim 1, wherein the compounds are 5-amino[1,2,4]thiadiazole derivatives substituted in positions 3 and 5, for example, 5-amino-3-(2-nitroxypropyl)-1,2,4-thiadiazole compounds, having the general formula: 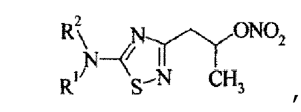 wherein
R¹ and R² may be the same or different and independently represent hydrogen, substituted or unsubstituted aryl or heteroaryl, or aralkyl, alkyl, cycloalkyl, as well as R¹ together with R² may represent heterocyclyl (optionally substituted morpholine, piperazine, and piperidine).

3. The compounds according to claim 1, wherein the compounds are 5-amino-[1,2,4]thiadiazole derivatives substituted in positions 3 and 5, for example, 5-amino-3-(2-aminopropyl)-1,2,4-thiadiazole compounds, having the general formula: 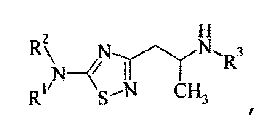 wherein
R¹, R², and R³ may be the same or different and independently represent hydrogen, alkyl, aryl, aralkyl, heteroaryl, heteroaralkyl, cycloalkyl, as well as R¹ together with R² may represent heterocyclyl (for example, optionally substituted pyrrolidine, piperidine, azepane, piperazine, morpholine, etc.) provided that, when R¹ is H, then R² is other than hydrogen or methyl.
